# EUROPÄISCHE PATENTSCHRIFT

(11) **EP 1 968 960 B1**
(45) Veröffentlichungstag und Bekanntmachung des Hinweises auf die Patenterteilung: **26.05.2010**
(21) Anmeldenummer: 06830698.4
(22) Anmeldetag: 18.12.2006
(51) Int. Cl.: C07D 295/02

(54) **RACEMATSPALTUNG VON 2,6-TRANS-DIMETHYLMORPHOLIN**
RACEMIC SEPARATION OF 2,6-TRANS-DIMETHYLMORPHOLINE
SEPARATION RACEMIQUE DE 2,6-TRANS-DIMETHYLMORPHOLINE

(30) Priorität: 30.12.2005 DE 102005063192
(43) Veröffentlichungstag der Anmeldung: 17.09.2008
(73) Patentinhaber: BASF SE, 67056 Ludwigshafen (DE)
(72) Erfinder: DITRICH, Klaus, 67161 Gönnheim (DE); HAESE, Frank, 63128 Dietzenbach (DE)
(74) Vertreter: Dörper, Thomas Michael
(86) Internationale Anmeldenummer: PCT/EP2006/069862
(87) Internationale Veröffentlichungsnummer: WO 2007/077118

(56) Entgegenhaltungen:
- WO-A-94/27966
- DE-A1- 2 822 326
- US-A- 4 571 424
- US-A1- 2004 039 206
- FALBE J ET AL: "RÖMPP CHEMIE LEXIKON, Mandelsäure" ROMPP CHEMIE LEXIKON. M-PK, 4. AUFLAGE, STUTTGART, THIEME VERLAG, DE, Bd. 4, 1998, Seiten 2516-2517, XP002439538
- ULLMANN AND GERHARTZ ED: "Ullmann's encyclopedia of Industrial Chemistry B2" ULLMANN'S ENCYCLOPEDIA OF INDUSTRIAL CHEMISTRY. 4. AUFL. BAND 17, WEINHEIM, VCH VERLAG, DE, Bd. 17, 1980, Seiten 451-457, XP002439539
- EUGEN MÜLLER (ED.): "Houben-Weyl, Methoden der organischen Chemie (vierte Auflage), C, N-Doppelbindungs-systeme" HOUBEN-WEYL METHODEN DER ORGANISCHEN CHEMIE, GEORGE THIEME VERLAG, STUTTGART, DE, Bd. IV, 1955, Seiten 508-519, XP002439540
- WILEN S H ET AL: "Tetrahedron report number 38: Strategies in optical resolutions" TETRAHEDRON, ELSEVIER SCIENCE PUBLISHERS, AMSTERDAM, NL, Bd. 33, Nr. 21, 1977, Seiten 2725-2736, XP002293732 ISSN: 0040-4020
- EMANUELA LICANDRO, STEFANO MAIORANA, CLARA BALDOLI, LAURA CAPELLA, DARIO PERDICCIA: "Enantioselective synthesis of (R)-(-)-baclofen using Fischer-type carbene anions" TETRAHEDRON: ASYMMETRY, Bd. 11, 2000, Seiten 975-980, XP002439541

## Beschreibung

Die vorliegende Erfindung betrifft ein Verfahren zur Herstellung von optisch aktivem trans-2,6-Dimethylmorpholin durch Umsetzen von racemischem trans-2,6-Dimethylmorpholin mit optisch aktiver Mandelsäure in Gegenwart von Essigsäure.

### Stand der Technik

Trans-2,6-Dimethylmorpholin ist ein Zwischenprodukt für die Synthese von Fungiziden und Arzneimitteln. Da jedoch in der Regel nur ein Enantiomer des trans-2,6-Dimethylmorpholins die gesuchte biologische Aktivität aufweist, ist es wünschenswert trans-2,6-Dimethylmorpholin in optisch aktiver Form rein darzustellen.

Dies erfolgt beispielsweise durch eine aufwändige enantioselektive Synthese wie sie von Licandro et al. (Gazzetta Chimica Italiana 127, 815-817, 1997) beschrieben wird. Dort wird (-)-(2R,6R)-2,6-Dimethylmorpholin in einer neunstufigen Synthese aus 1,2-Propandiol aufgebaut.

Diese Herstellung ist sehr kosten- und zeitaufwändig; demzufolge besteht ein großer Bedarf an alternativen Herstellungsmethoden, die einen billigeren und schnelleren Zugang zu optisch aktivem trans-2,6-Dimethylmorpholin gestatten.

### Beschreibung der Erfindung

Die vorliegende Erfindung betrifft ein Verfahren zur Herstellung von optisch aktivern trans-2,6-Dimethylmorpholin durch (i) Umsetzen von pro mol racemischem trans-2,6-Dimethylmorpholin mit bis zu 0,5 mol optisch aktiver Mandelsäure und bis zu 0,5 mol Essigsäure, (ii) Abtrennen des gebildeten Salzes aus optisch aktiver Mandelsäure und dem einen Enantiomer des trans-2,6-Dimethylmorpholin von dem anderen Enantiomer des trans-2,6-Dimethylmorpholin und (iii) Isolierung des gewünschten optisch aktiven trans-2,6-Dimethylmorpholin.

Racemisches trans-2,6-Dimethylmorpholin lässt sich aus einem Gemisch von trans- und cis- 2,6-Dimethylmorpholin, wie es durch dem Fachmann bekannte Synthesen erhältlich ist, durch Destillation isolieren.

Das racemische trans-2.6-Dimethylmorpholin wird dann mit optisch aktiver Mandelsäure umgesetzt. Pro Mol racemischem trans-2,6-Dimethylmorpholin werden bis zu bis zu 0,5 Mol optisch aktiver Mandelsäure zugesetzt. Die Mandelsäure kann zu dem trans-2,6-Dimethylmorpholin zugegeben werden oder umgekehrt. Bevorzugt ist die Vorlage der Mandelsäure und die Zugabe von racemischem trans-2,6-Dimethylmorpholin.

Mit D-Mandelsäure kristallisiert das S,S-trans-2,6-Dimethylmorpholin, mit L-Mandelsäure kristallisiert das R,R-trans-2,6-Dimethylmorpholin. Die Struktur des auskristallisierenden Amins wurde per Röntgenstrukturanalyse bestimmt.

Die besondere Ausgestaltung des erfindungsgemäßen Verfahrens besteht darin, dass pro Mol racemischem trans-2,6-Dimethylmorpholin bis zu 0,5 Mol optisch aktiver Mandelsäure und bis zu 0,5 Mol Essigsäure zugesetzt werden.

Diese Verfahrensweise hat den Vorteil, dass ein Enantiomer des trans-2,6-Dimethylmorpholin mit der optisch aktiven Mandelsäure das entsprechende Salz bildet, das aus dem Reaktionsmedium ausfällt, während das andere Enantiomer des trans-2,6-Dimethylmorpholin zusammen mit der Essigsäure, in Lösung verbleibt. Dadurch lassen sich die Enantiomeren leicht voneinander trennen und isolieren.

Die Temperatur des erfindungsgemäßen Verfahrens ist nicht besonders kritisch. Sie kann in einem weiten Bereich variiert werden. Bevorzugt ist eine Temperatur zwischen 0 und 150, besonders bevorzugt zwischen 5 und 50°C. Gewünschtenfalls kann die Reaktionsmischung gekühlt werden um entstehende Mischungswärmen abzuführen und /oder Ausfällungen von Salzen zu komplettieren.

Die Reaktion kann in vielen gängigen organischen Lösungsmitteln durchgeführt werden; bevorzugt werden Alkanole als Lösungsmittel eingesetzt. Mit besonders guter Ausbeute verläuft das erfindungsgemäße Verfahren mit Isopropanol als Lösungsmittel.

Die Abtrennung des in Schritt (i) entstandenen Salzes erfolgt bevorzugt über Auskristallisieren und Filtration der Kristalle. Zum Beschleunigen des Auskristallisierens empfiehlt es sich, einige Impfkristalle zuzugeben. Die entstandenen Kristalle haben in der Regel schon eine hohe optische Reinheit von über 70, bevorzugt über 80% ee. Um eine weitere Erhöhung der optischen Reinheit zu erreichen, können diese Salze umkristallisiert werden. Beispielsweise liefert eine zweimalige Umkristallisierung der Salze aus Isopropanol eine optische Reinheit von über 98%ee.

Aus dem Salz kann das optisch aktive R,R-trans-2,6-Dimethylmorpholin durch Zugabe einer Base, bevorzugt Natronlauge freigesetzt und beispielsweise durch Destillation im Vakuum isoliert werden.

Das optisch aktive S,S-trans-2,6-Dimethylmorpholin kann aus der Reaktionslösung als Salz isoliert und ebenfalls mittels Base freigesetzt werden.

Unter geeigneten Bedingungen gelingt es, auch das zur Racematspaltung eingesetzte Hilfsreagenz - die Mandelsäure - in optisch aktiver Form zurückzugewinnen.

Die folgenden Beispiele dienen der weiteren Veranschaulichung und Beschreibung der Erfindung.

### Experimenteller Teil

Im folgenden ist das generelle Schema des erfindungsgemäßen Verfahrens abgebildet

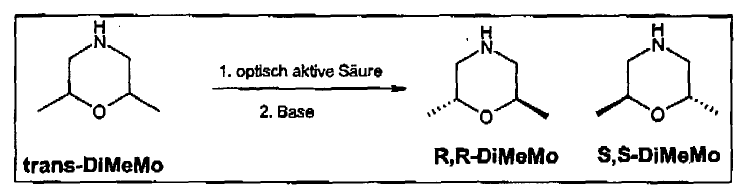

### Racematspaltung im präparativen Maßstab

### a. Fällen des R-Mandelats

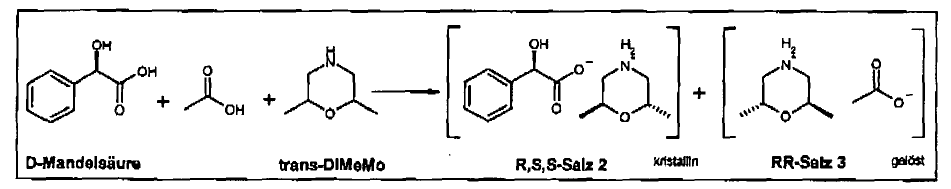

### Durchführung:

D-Mandelsäure (330.4 g, 2.174 mol) wurde in Isopropanol (2 L) vorgelegt, zunächst mit Essigsäure (130.4 g, 2.174 mol) und dann zügig tropfenweise mit trans-2,6-dimethylmorpholin (**trans-DiMeMo**, 500 g, 4.348 mol) versetzt. Die Temperatur der Mischung stieg dabei auf 45°C. Die klare Lösung wurde mit **R,S,S-Salz 2** angeimpft und über Nacht bei Raumtemperatur stehengelassen. Anderntags wurde das Gemisch unter Rühren auf 10°C abgekühlt und der ausgefallene Rückstand abgesaugt. Das im isolierten (**R,S,S-Salz 2**. 400 g (feucht), 34%) gebundenene **S,S-DIMeMo** wies eine optische Reinheit von 74%ee auf. Das Salz wurde in Isopropanol (1 L) aufgekocht, auf Raumtemperatur abgekühlt und mit einem Kristall **R,S.S-Salz 2** angeimpft. Man ließ wieder über Nacht bei Raumtemperatur stehen und saugte andemtags erneut ab. Man erhielt 350 g (30%, feucht) **R,S,S-Salz 2**, das darin gebundene **S,S-DiMeMo** wies eine optische Reinheit von 97.7%ee auf. Das Salz wurde nochmals in Isopropanol (1 L) warm (70°C) gelöst und über Nacht bei Raumtemperatur stehengelassen. Wieder wurde mit einem Kristall **R,S,S-Salz 2** angeimpft und über Nacht stehengelassen. Das ausgefallene Salz wurde abgesaugt und im Trockenschrank getrocknet. Man erhielt 266.5 g (23%) **R,S,S-Salz 2**, das darin gebundene **S,S-DiMeMo** wies eine optische Reinheit von 98.3%ee auf. Der Schmelzpunkt vom **R,S,S-Salz 2** lag bei 134°C.

Die absolute Konfiguration des im Salz gebundenen trans-2,6-Dimethylmorpholins wurde durch Röntgenstrukturanalyse bestimmt (siehe Anlage 1). Danach fällt mit D-Mandelsäure bevorzugt das **S,S-DIMeMo**.

### b. Freisetzung des S,S-2,6-Dimethylmorpholins (S,S-DiMeMo) aus dem D-Mandelat (R,S,S-Salz 2) im Basischen

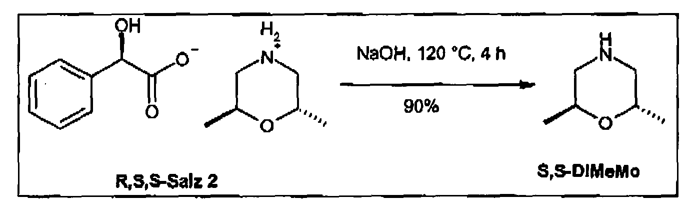

### Durchführung:

Das D-Mandelat (**R,S,S-Salz 2**, 266.5 g, 1 mol) wurde in 20%iger Natronlauge (400 mL) gelöst und eine Stunde bei 50°C gerührt. Danach resultierte eine klare Lösung. Man erhöhte die Badtemperatur auf 120°C und destillierte über eine Destillationsbrücke das freigesetzte **S,S-DiMeMo** im Azeotrop mit Wasser ab (Übergang: 100°C). Nachdem ca. 300 mL Kondensat übergegangen waren, kühlte man die Destillationsvorlage auf Raumtemperatur ab und säuerte mit 10%iger HCl an. Die ausgefallene Mandelsäure wurde abgesaugt und im Vakuum getrocknet. Eine Analyse der optischen Reinheit ergab, dass die D-Mandelsäure vollständig racemisiert war.

Das übergegangene Kondensat wurde durch Zugabe von fester NaOH gesättigt; es schied sich eine organische Phase ab, die mit MTBE (200 mL) verdünnt wurde. Man extrahierte mit MTBE (2 x 100 mL), vereinigte die Extrakte, trocknete über Na₂SO₄ und rotierte ein. Man erhielt 101 g Rohprodukt, das im Wasserstrahlpumpenvakuum fraktioniert wurde. Das reine **S.S-DiMeMo** ging bei 37-39 °C/14 mm über. Man erhielt 88 g (18% bezogen auf eingesetztes Racemat **trans-DiMeMo**) **S,S-DIMeMo** als klares Öl.

Drehwert [α]_{D} = -6.15 ° (pur, d= 0.94 g*cm⁻³).
Laut GC-Analyse lag die optische Reinheit des isolierten **S,S-DiMeMos** bei 97.9%ee.

### c. Fällen des S-Mandelats

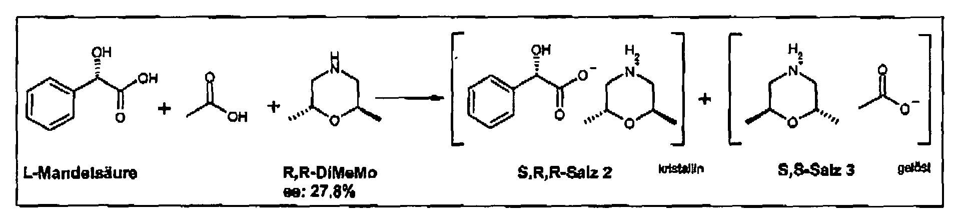

### Durchführung:

Aus den Mutterlaugen von dem in a. gewonnenem **R,S,S-Salz 2** wurde nach dem in b, geschildertem Verfahren ein **R,R-DiMeMo** mit einer optischen Reinheit von 27.8%ee (**S,S : R,R** = 36 : 64) freigesetzt Insgesamt erhielt man 312 g (74%) dieser Mischung.

L-Mandelsäure (312.5 g, 2.06 mol) und Essigsäure (69.7 g,1.16 mol) wurden in Isopropanol (1500 mL) vorgelegt und zügig mit trans-2,6,Dimethylmorpholin (370 g, 3.21 mol, **R,R : S,S** = 64 : 36) versetzt. Die Temperatur des Gemisches stieg dabei bis ca. 40°C. Beim Abkühlen auf Raumtemperatur begann das **S,R,R-Salz 2** auszufallen. Man ließ über Nacht bei Raumtemperatur stehen und saugte anderntags das ausgefallene Salz ab. Man erhielt 400 g (47% bezogen auf eingesetztes Startmaterial) feuchtes **S,R,R-Salz 2.** Das im **S,R,R-Salz 2** gebundene **R,R-DiMeMo** wies eine optische Reinheit von 81.5%ee auf. Das Salz wurde in Isopropanol (1 L) gelöst und über Nacht bei Raumtemperatur stehengelassen. Wiederum wurde das **S,R,R-Salz 2** abgesaugt, man erhielt 320 g **S,R,R-Salz 2**, das darin gebundene **R,R-DiMeMo** wies eine optische Reinheit von 95.6%ee auf. Das so gewonnene Salz wurde nochmals aus Isopropanol (900 mL) umkristallisiert, abgesaugt und im Vakuum getrocknet.

Man erhielt 274 g (50% bezogen auf eingesetztes R,R-Dimethylmorpholin **R,R-DimeMo**). Das darin gebundene R,R-DiMeMo hatte eine optische Reinheit von 99.1 %ee.

### d. Freisetzung des R,R-2,6-Dimethylmorpholins (R,R-DiMeMo) aus dem L-Mandelat (S,R,R-Salz 2) im Sauren

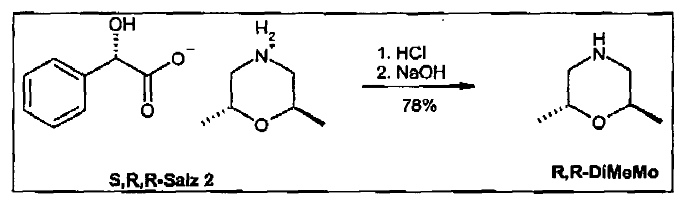

### Durchführung:

Das L-Mandelat (**S,R,R-Salz 2,** 274 g, 1.03 mol) wurde in Wasser (400 mL) gelöst und durch Zugabe von konzentrierter Salzsäure auf pH 1.5 gebracht. Man kühlte auf 1°C und impfte durch Zugabe einiger Kristalle von S-Mandelsäure an. Man rührte 2 Stunden bei 0°C nach und saugte den ausgefallenen Kristallbrei ab. Der Filterrückstand wurde mit Eiswasser (100 mL) gewaschen und im Vakuum bei 50°C getrocknet. Man erhielt 86 g (57%) L-Mandelsäure, die It. Analyse optisch rein war.
Die vereinten Filtrate wurden unter Eiskühlung durch Zugabe fester NaOH basisch (pH 14) gestellt. Bei Zugabe von t-Butyl-methylether (300 mL) fiel ein weißer, schmieriger Feststoff aus. Man dekantierte die organische Phase ab und rührte noch dreimal mit t-Butyl-methylether (3 mal 100 mL) aus. Die organischen Phasen wurden jeweils abdekantiert und die vereinten organischen Extrakte über Natriumsulfat getrocknet. Das Lösungsmittel wurde im Vakuum abgezogen und der Rückstand im Vakuum fraktioniert. Das reine **R,R-DiMeMo** ging bei 36°C/13 mm über. Man erhielt 93.2 g (18.5% bezogen auf eingesetztes Racemat **trans-DiMeMo) R,R-DiMeMo** als klares Öl.
Drehwert [α]_{D} = 6.28 ° (pur, d= 0.94 g*cm⁻³).
Laut GC-Analyse lag die optische Reinheit des isolierten **R,R-DiMeMos** bei 99.1%ee.

### Racematspaltung von 2,6-trans-Dimethylmorpholin

### Zusammenfassung

Verfahren zur Herstellung von optisch aktivem trans-2.6-Dimethylmorpholin durch (i)Umsetzen von racemischem trans-2,6-Dimethylmorpholin mit D-Mandelsäure, (ii) Abtrennen des gebildeten Salzes aus D-Mandelsäure und dem einen Enantiomer des trans-2,6-Dimethylmorpholin von dem anderen Enantiomer des trans-2,6-Dimethylmorpholin und (iii) Isolierung des gewünschten optisch aktiven trans-2,6-Dimethylmorpholin.

## Patentansprüche

1. Verfahren zur Herstellung von optisch aktivem trans-2,6-Dimethylmorpholin durch (i) Umsetzen von pro mol racemischem trans-2,6-Dimethylmorpholin mit bis zu 0.5 mol optisch aktiver Mandelsäure und bis zu 0,5 mol Essigsäure, (ii) Abtrennen des gebildeten Salzes aus Mandelsäure und dem einen Enantiomer des trans-2,6-Dimethylmorpholin von dem anderen Enantiomer des trans-2,6-Dimethylmorpholin und (iii) Isolierung des gewünschten optisch aktiven trans-2,6-Dimethylmorpholin.

2. Verfahren nach Anspruch 1, **dadurch gekennzeichnet, dass** die Abtrennung (ii) durch Ausfällen des Salzes aus dem Reaktionsmedium erfolgt.

3. Verfahren nach Anspruch 1, **dadurch gekennzeichnet, dass** die Reaktion in Isopropanol durchgeführt wird.

4. Verfahren nach Anspruch 1-3, **dadurch gekennzeichnet, dass** man mit D-Mandelsäure das S,S-trans-2,6-Dimethylmorpholin ausfällt

5. Verfahren nach Anspruch 1-3, **dadurch gekennzeichnet, dass** man mit L-Mandelsäure das R,R-trans-2,6-Dimethylmorpholin ausfällt.

## Claims

1. A process for preparing optically active trans-2,6-dimethylmorpholine by (i) reacting each mole of racemic trans-2,6-dimethylmorpholine with up to 0.5 mol of optically active mandelic acid and up to 0.5 mol of acetic acid, (ii) removing the salt formed from mandelic acid and one enantiomer of trans-2,6-dimethylmorpholine from the other enantiomer of trans-2,6-dimethylmorpholine and (iii) isolating the desired optically active trans-2,6-dimethylmorpholine.

2. The process according to claim 1, wherein the removal (ii) is effected by precipitating the salt out of the reaction medium.

3. The process according to claim 1, wherein the reaction is carried out in isopropanol.

4. The process according to claim 1-3, wherein the S,S-trans-2,6-dimethylmorpholine is precipitated with D-mandelic acid.

5. The process according to claim 1-3, wherein the R,R-trans-2,6-dimethylmorpholine is precipitated with L-mandelic acid.

## Revendications

1. Procédé pour la préparation de *trans*-2,6-diméthylmorpholine optiquement active par (i) mise en réaction avec, par mole de *trans*-2,6-diméthylmorpholine racémique, jusqu'à 0,5 mole d'acide mandélique optiquement actif et jusqu'à 0,5 mole d'acide acétique (ii) séparation du sel formé d'acide mandélique et de l'un des énantiomères de la *trans-2,6-*diméthylmorpholine d'avec l'autre énantiomère de la *trans*-2,6-diméthylmorpholine et (iii) isolement de la *trans*-2,6-diméthylmorpholine optiquement active recherchée.

2. Procédé selon la revendication 1, **caractérisé en ce que** la séparation (iii) s'effectue par précipitation du sel à partir du mélange réactionnel.

3. Procédé selon la revendication 1, **caractérisé en ce que** la réaction est effectuée dans de l'isopropanol.

4. Procédé selon la revendication 1-3, **caractérisé en ce qu'**on fait précipiter la S,S-*trans*-2,6-diméthylmorpholine avec de l'acide D-mandélique.

5. Procédé selon la revendication 1-3, **caractérisé en ce qu'**on fait précipiter la R,R-*trans*-2,6-diméthylmorpholine avec de l'acide L-mandélique.
